## Europäisches Patentamt

## European Patent Office

## Office européen des brevets

(19)

(11) Veröffentlichungsnummer: **0 086 960**
**B1**

(12) **EUROPÄISCHE PATENTSCHRIFT**

(45) Veröffentlichungstag der Patentschrift:
04.09.85

(51) Int. Cl.⁴: **A 61 F 13/10**

(21) Anmeldenummer: **83100512.9**

(22) Anmeldetag: **21.01.83**

(54) **Tennisarm-Bandage.**

(30) Priorität: **23.02.82 DE 3206454**

(43) Veröffentlichungstag der Anmeldung:
**31.08.83 Patentblatt 83/35**

(45) Bekanntmachung des Hinweises auf die Patenterteilung:
**04.09.85 Patentblatt 85/36**

(84) Benannte Vertragsstaaten:
**AT BE CH DE FR GB IT LI NL SE**

(56) Entgegenhaltungen:
**DE - A - 2 632 706**
**US - A - 3 877 426**
**US - A - 3 942 525**
**US - A - 4 014 327**
**US - A - 4 048 991**
**US - A - 4 243 028**
**US - A - 4 299 214**

(73) Patentinhaber: **Beiersdorf Aktiengesellschaft,
Unnastrasse 48, D-2000 Hamburg 20 (DE)**

(72) Erfinder: **Müller, Peter, Richard-Wagner-Strasse 2,
D-5276-Wiehl (DE)**
Erfinder: **Nägelen, Joachim, Seevestrasse 22,
D-2105 Seevetal 3 (DE)**

ACTORUM AG

# Beschreibung

Der beim Tennisspielen oftmals beiden Tennisspielern auftretende Tennisarm, auch als Epicondylitis bezeichnet, wird nach den verschiedensten Methoden behandelt. Neben der Behandlung durch Spritzen im Bereich der Epicondylenen mit einem geeigneten pharmazeutischen Präparat mit der Folge einer kurzfristigen Schmerzfreiheit ist die Anwendung einer speziellen Massage bekannt, die zu einer spürbaren Besserung und sogar zu einer Schmerzfreiheit führen kann. Diese letztgenannte Behandlungsmethode ist jedoch von Laien selbst nicht anwendbar und muss daher zu ihrer wirksamen Durchführung von einem mit der Methode vertrauten Fachmann durchgeführt werden.

Neben der medikamentösen Behandlung und der Anwendung von Massagen sind Bandagen bekannt, die eine wirksame Selbstbehandlung des Tennisarmes ermöglichen bzw. prophylaktisch wirken sollen.

So ist nach dem DE-U-7622988 eine Tennisarm-Bandage bekannt, bei der die den Unterarm umschliessende Bandage ein Feld von in einer Vielzahl in Abstand nebeneinander angeordneten länglichen Körpern aus elastischem Werkstoff aufweist, so dass die über den Unterarm gezogene Bandage sich mit ihren elastischen Körpern im Bereich entweder der äusseren oder inneren Epicondylenen fest an den Unterarm anlegen kann. Durch die elastischen Körper der Bandage soll bei einem Bewegen des Armes eine Massage auf die Unterarmmuskulatur ausgeübt werden, um einem Verkrampfen und Verhärten der Unterarmmuskulatur entgegenzutreten bzw. die verkrampfte und verhärtete Unterarmmuskulatur aufzulockern.

Mit dieser bekannten Epicondylitis-Bandage ist der auf die Muskel-Sehnen-Ansätze wirkende Druck nicht regulierbar, so dass keine ausreichende Entlastung des schmerzenden Bereiches erfolgt.

Durch das DE-U-7837412 ist eine Unterarm-Ellenbogen-Bandage bekanntgeworden, die durch ein um den Arm legbares und mit einem Klettenverschluss zu sicherndes elastisches Grundband, durch wenigstens einen an der Aussenseite des Grundbandes befestigten, unelastischen, mit seinem freien Ende mittels eines Klettenverschlusses an dem Grundband festzulegenden Streifen und durch ein an dem Grundband befestigtes elastisches Deckband, das über den wenigstens einen unelastischen Streifen zu legen und mittels eines Klettenverschlusses an seinem freien Ende an dem Grundband festzulegen ist, gekennzeichnet ist. Eine derartige Bandage soll besonders für Tennisspieler zur Stützung ihres Tennisarmes oder auch zur Stützung eines Armes, der die Tennisarmkrankheit hat, geeignet sein. Auch bei dieser bekannten Bandage ist ein individuell regulierbarer Druck auf die Muskel-Sehnen-Ansätze nicht möglich. Darüber hinaus wirkt die Bandage nicht auf den ganzen Unterarmmuskel und übt darüber hinaus keine Massagewirkung aus.

Darüber hinaus sind Schutzvorrichtungen für Arm und Hand in den verschiedensten Ausführungsformen bekannt.

So beschreibt die DE-A-2460582 eine Schutzvorrichtung für Arm und Hand für die Verwendung bei sportlichen Wettkämpfen, die aus einer Hülle besteht, die ein elastisches Schaumstoffmaterial umgibt und die im wesentlichen so ausgebildet ist, dass sie an Daumen, Hand, Handgelenk, Arm und Ellenbogen einer Person getragen werden kann und Befestigungsmittel hierfür aufweist. Diese bekannte, einen prothesenartigen Eindruck vermittelnde Schutzvorrichtung übt aufgrund ihrer Ausgestaltung keine Oberflächenmassage auf den Unterarm aus; auch ein regulierbarer Druck kann mit dieser Schutzvorrichtung nicht auf die Muskel-Sehnen-Ansätze ausgeübt werden. Eine Streckung der Unterarmmuskeln zur Entlastung des schmerzenden Bereiches ist mit dieser Schutzvorrichtung nicht erzielbar.

Eine Handgelenkstütze, die wahlweise an der rechten oder der linken Hand tragbar ist, bestehend aus einem um das Handgelenk festlegbaren, biegsamen Band mit einer Daumenöffnung, von der sich nach beiden Seiten Bandteile unterschiedlicher Länge wegerstrecken, wobei an dem Ende des längeren Bandteiles ein erster Verschlussteil angeordnet ist, der an einem an anderer Stelle des Bandes angeordneten zweiten Verschlussteil verstellbar festlegbar ist, ist durch die DE-A-1578667 bekannt. Bei dieser Handgelenkstütze bilden die beiden Bandteile einen stumpfen Winkel miteinander, wobei der zweite Verschlussteil ebenfalls an dem längeren Bandteil angeordnet ist. Damit soll eine Handgelenkstütze geschaffen sein, die sich an unterschiedliche Handgrössen glatt anlegt und die Mittelhand mit umfasst. Eine derartige Handgelenkstütze ist jedoch nicht zur Behandlung von Tennisarmen geeignet.

Es ist ferner nach der US-A-4014327 eine Tennisarm-Bandage bekannt, die aus zwei Zugbändern besteht, die über einen an der Innenseite des Unterarmes anliegenden Steg mit einer metallischen Einlage verbunden sind. Mit einer derartig ausgebildeten, prothesenartigen Tennisarm-Bandage ist jedoch eine Druckausübung auf den ganzen Unterarmmuskel nicht möglich, denn es wird nur eine Kompressionswirkung am oberen Muskel-Sehnen-Ansatz und eine nutzlose Kompression unterhalb des Handgelenkes erzielt.

Demgegenüber ist es Aufgabe der vorliegenden Erfindung, eine Tennisarm-Bandage mit einer individuell vornehmbaren Druckregulierung zur Stützung und Entlastung des beanspruchten Armgelenkes, insbesondere von Tennisspielern, zu schaffen, mit der eine gleichmässige Kompression bei gleichzeitiger optimaler Anpassung an das Armgelenk und eine gute Massagewirkung erzielbar ist.

Zur Lösung dieser Aufgabe wird eine Tennisarm-Bandage vorgeschlagen, die den Unterarm über seine ganze Länge umschliesst und aus einer schlauchförmigen Umhüllung aus einem quer- und längselastischen Gewebe oder Gewirke besteht und dadurch gekennzeichnet ist, dass die Umhüllung benachbart zu ihrem einen, zum Ellbogen weisenden Ende ein auf dem Schmerzzentrum unterhalb der Armbeuge zu liegen bestimmtes, mit der Umhüllung fest verbundenes und in seiner Länge verstellbares erstes Zugband und benachbart zu ihrem anderen, zum Handgelenk weisenden Ende ein zweites Zugband aufweist, das an der Umhüllung lösbar und abstandsveränderlich von dem ersten Zugband befestigt und in seiner Länge veränderbar ausgebildet ist.

Aufgrund der erfindungsgemässen Ausgestaltung ist eine Tennisarm-Bandage geschaffen, bei der das bielastische, dauerhaft aktive Material für eine gleichmässige Kompression und optimale Anpassung an das Gelenk sorgt. Durch die Möglichkeit, die Zugbänder stufenlos regulieren zu können, kann je nach Einstellung die Kompression noch verstärkt werden. Im Gegensatz zu herkömmlichen Bandagen übt eine derart ausgebildete Tennisarm-Bandage nicht nur auf die Muskel-Sehnen-Ansätze — Schmerzzentrum — einen individuell regulierbaren Druck aus, sondern auf den ganzen Unterarmmuskel. Durch den ausgeübten Druck wird der Unterarmmuskel im ganzen gestreckt — er wird länger — und entlastet damit den schmerzenden Bereich. Durch die Quer- und Längselastizität des verwendeten Bindenmaterials wird gleichzeitig eine gute Oberflächenmassage erzielt. Eine Druckeinwirkung auf den Unterarm wird nicht nur in horizontaler, sondern auch in vertikaler Richtung erreicht, da die schlauchförmige Umhüllung der Tennisarm-Bandage in beiden Richtungen ständig unter Zug steht, so dass gerade bei der Durchführung von Bewegungen die Massagewirkung wesentlich erhöht wird.

Die aus der bielastischen Umhüllung bestehende Tennisarm-Bandage wird über die ganze Länge des Unterarmes gezogen, so dass das mit der Umhüllung fest verbundene Zugband auf dem Schmerzzentrum unterhalb der Armbeuge liegt, wobei das der Armbeuge zugekehrte Ende der schlauchförmigen Umhüllung wahlweise über das Gelenk gezogen werden kann und somit eine zusätzliche Abstützung erzielt wird. Es besteht jedoch auch die Möglichkeit, bei einer entsprechenden Abstandsanordnung dieses Zugbandes vom Hüllenende den dann erhaltenen Umhüllungsabschnitt über das Zugband zu legen, so dass dieses gleichzeitig mit verdeckt wird. Das handgelenkseitige und lösbar mit der schlauchförmigen Umhüllung verbundene Zugband wird entsprechend der Armlänge so auf der schlauchförmigen Umhüllung bei angelegter Tennisarm-Bandage fixiert, dass dieses Zugband auf dem Handgelenk bzw. oberhalb des Handgelenkes zu liegen kommt. Auch das dann hier evtl. überstehende Ende der schlauchförmigen Umhüllung kann nach Anziehen des Zugbandes so auf das Zugband umgeschlagen werden, dass dieses verdeckt ist.

Bei angelegter Tennisarm-Bandage erfolgt ein intensives Zusammenwirken von längsgerichteter Kompression der unter Zug stehenden schlauchförmigen Umhüllung mit einer quergerichteten Kompression der Zugbänder in Verbindung mit der schlauchförmigen Umhüllung.

Weitere vorteilhafte Ausgestaltungen der Erfindung gehen aus den Unteransprüchen hervor.

In der Zeichnung ist der Gegenstand der Erfindung an einem Ausführungsbeispiel dargestellt, und zwar zeigt

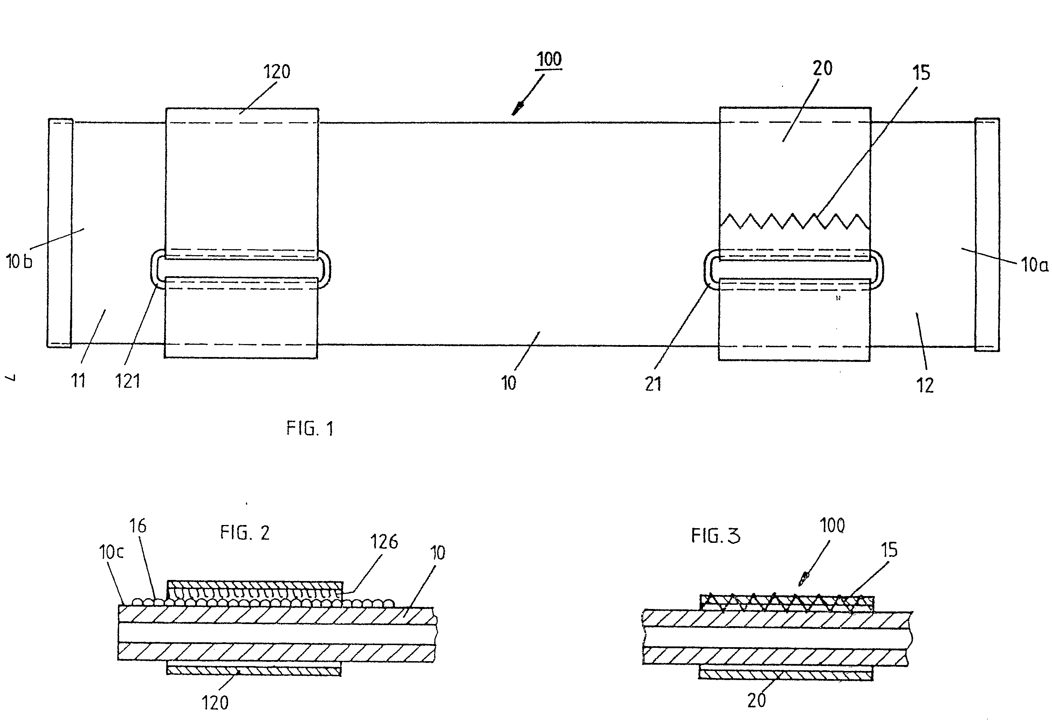

. Fig. 1 die Tennisarm-Bandage in einer Ansicht von oben und

Fig. 2 und Fig. 3 die beiden die Zugbänder tragenden Endabschnitte der Tennisarm-Bandage in einem vergrösserten senkrechten Teilschnitt.

Die in Fig. 1 dargestellte und mit 100 bezeichnete Tennisarm-Bandage besteht aus einer schlauchförmigen Umhüllung 10 als tragender Teil für zwei in den Endbereichen 10a und 10b an der Umhüllung angeordnete Zugbänder 20, 120.

Die schlauchförmige Umhüllung 10 weist mindestens Unterarmlänge auf und besteht aus einem längs- und querelastischen Gewirke oder Gestricke aus Fäden aus Baumwolle od. dgl., Polyamid und aus elastisch vernetztem Polyurethan. Die Umhüllung 10 kann auch aus einem längs- und querelastischen Frotteegewebe oder -gewirk bestehen. Die Verwendung eines Frotteegewebes oder -gewirkes erbringt den Vorteil einer guten Feuchtigkeitsaufnahme, wobei darüber hinaus eine derart ausgebildete Umhüllung 10 auch noch frottierende Eigenschaften besitzt. Die Porosität des Gewirkes oder Gestrickes sorgt dafür, dass kein Feuchtigkeitsstau oder Wärmestau zwischen der Haut des mit der Bandage geschützten Körperteiles und der Bandage selbst entstehen kann. Vorteilhafterweise weist die Umhüllung 10 eine Länge auf, die gegenüber der Unterarmlänge grösser ist, so dass nach dem Überziehen der Tennisarm-Bandage auf den Unterarm die Bandage mit einem Abschnitt über den Ellbogen hinaus in den Oberarmbereich reicht.

Das benachbart zum freien und bei angelegter Bandage dem Ellbogen des zu schützenden Armes zugekehrte Ende 10a der schlauchförmigen Umhüllung 10 angeordnete Zugband 20 ist mit einer in Umhüllungslängsrichtung verlaufenden Naht 15 an der Umhüllung 10 derart befestigt, dass eine Längenverstellbarkeit des Zugbandes 20 nicht beeinträchtigt wird.

Das weitere Zugband 120 ist an dem anderen Ende 10b der Umhüllung 10 derart lösbar auf der Umhüllung 10 befestigt, dass eine Abstandsveränderung von dem Zugband 20 vorgenommen werden kann, damit das Zugband 120 jeweils im Bereich des Handgelenkes oder vor dem Handgelenk zur Anlage gebracht werden kann. Diese lösbare Befestigung des Zugbandes 120 auf der schlauchförmigen Umhüllung 10 erfolgt mittels eines in an sich bekannter Weise ausgebildeten Klettenverschlusses. Hiernach weist das Zugband 120 auf seiner der Aussenfläche 10c der Umhüllung 10 zugekehrten Fläche einen Klettenabschnitt 126 auf, der das eine Verschlusteil des Klettenverschlusses bildet und aus im Abstand voneinander angeordneten aufwärts gerichteten Häkchen besteht. Das andere Verschlusteil des Klettenverschlusses ist auf der Aussenfläche 10c der Umhüllung 10 in Form eines Abschnittes mit einer Schlaufenausbildung befestigt. Dieser mit 16 bezeichnete Abschnitt besteht aus einer Gruppe relativ dünner Polfasern, in die zur Bildung des Klettenverschlusses die aufwärts gerichteten Häkchen des an dem Zugband 120 vorgesehenen Verschlussteiles eingreifen können (Fig. 2).

Die Anordnung der beiden Zugbänder 20, 120 an der schlauchförmigen Umhüllung 10 der Tennisarm-Bandage 100 ist derart, dass zu den freien Enden 10a, 10b der Umhüllung je ein Umhüllungsabschnitt 11 und 12 verbleibt. Dadurch ist die Möglichkeit gegeben, durch Umschlagen dieser Enden 11, 12 der Umhüllung 10 auf die Zugbänder 20, 120 diese zu verdecken, so dass im getragenen Zustand die Tennisarm-Bandage ein ansprechendes Aussehen aufweist.

Die beiden Zugbänder 20, 120 sind gleich ausgebildet, so dass nachstehend nur das Zugband 20 näher beschrieben wird.

Das Zugband 20 besteht aus einem unelastischen d.h. nicht dehnbaren Bandabschnitt, der an seinem einen freien Ende eine Schnalle 21 trägt, die aus einem rechteckförmigen Rahmenkörper aus Kunststoff gebildet ist. Bei dem Zugband 120 ist diese Schnalle mit 121 bezeichnet (Fig. 1).

Um das durch die Schnalle 21 gezogene freie Ende des Zugbandes 20 an diesem befestigen zu können, sind das Zugband und sein freier Endabschnitt zur Ausbildung eines an sich bekannten Klettenverschlusses gestaltet. Hierzu weist das Zugband 20 auf seiner Aussenfläche das eine Verschlussteil des Klettenverschlusses auf. Dieses Verschlussteil besteht aus auf der Zugbandaussenfläche vorgesehenen relativ dünnen Polfasern, während das andere Verschlussteil des Klettenverschlusses im Bereich des freien Endes des Zugbandes 20, und zwar auf der Zugbandseite vorgesehen ist, die auch das erstgenannte Verschlussteil mit den relativ dünnen Polfasern aufweist. Dieses Verschlussteil am freien Ende des Zugbandes 20 ist gebildet von einer Anzahl von im Abstand voneinander angeordneten, aufwärts gerichteten Häkchen oder Schlaufen, so dass nach dem Hindurchführen des freien Endes des Zugbandes 20 durch die Schnalle 21 und nach dem Anziehen des Zugbandes das freie Zugbandende gegen das auf der Aussenfläche des Zugbandes vorgesehene Verschlussteil in Form von relativ dünnen Polfasern aufgedrückt und somit an dem Zugband selbst gesichert ist.

Anstelle des vorangehend beschriebenen Klettenverschlusses für die beiden Zugbänder 20, 120 können auch in anderer Weise ausgebildete, nicht auftragende Verschlüsse zur Anwendung gelangen.

Die schlauchförmige Umhüllung 10 weist im Bereich ihrer beiden Enden 10a, 10b auf die Aussenseite der Umhüllung umgeschlagene Abschnitte auf, deren freie, umlaufende Ränder mit der Umhüllung vernäht sind, so dass bei angelegter Tennisarm-Bandage die Umhüllungsenden nicht in die Haut einschneiden können. Die schlauchförmige Umhüllung 10 weist einen gleichbleibenden, sich über die gesamte Länge der Umhüllung erstreckenden Durchmesser auf. Es besteht jedoch auch die Möglichkeit, eine zum

handgelenkseitigen Ende 10b konisch sich verjüngend ausgebildete schlauchförmige Umhüllung 10 als tragendes Teil für die beiden Zugbänder 20, 120 zu verwenden.

## Patentansprüche

1. Tennisarm-Bandage (100), die den Unterarm über seine ganze Länge umschliesst und aus einer schlauchförmigen Umhüllung (10) aus einem quer- und längselastischen Gewebe oder Gewirke besteht, dadurch gekennzeichnet, dass die Umhüllung (10) benachbart zu ihrem einen, zum Ellbogen weisenden Ende (10a) ein auf dem Schmerzzentrum unterhalb der Armbeuge zu Liegen bestimmtes, mit der Umhüllung (10) fest verbundenes und in seiner Länge verstellbares erstes Zugband (20) und benachbart zu ihrem anderen, zum Handgelenk weisenden Ende (10b) ein zweites Zugband (120) aufweist, das an der Umhüllung (10) lösbar und abstandsveränderlich von dem ersten Zugband (20) befestigt und in seiner Länge veränderbar ausgebildet ist.

2. Tennisarm-Bandage nach Anspruch 1, dadurch gekennzeichnet, dass die beiden Zugbänder (20, 120) unter Ausbildung freier Endabschnitte (11, 12) der Umhüllung (10) auf dieser angeordnet sind.

3. Tennisarm-Bandage nach den Ansprüchen 1 und 2, dadurch gekennzeichnet, dass jedes Zugband (20, 120) an seinem einen Ende eine Schnalle (21, 121) aus einem rechteckförmigen Rahmenkörper aus Kunststoff trägt, und dass das andere freie Ende des Zugbandes (20, 120) mittels eines Klettenverschlusses an dem Zugband (20, 120) selbst sicherbar ist, dessen eines Verschlussteil aus einem an dem freien Ende des Zugbandes und an dessen Aussenfläche vorgesehenen Abschnitt aus im Abstand voneinander angeordneten, aufwärts gerichteten Häkchen besteht, während das andere Verschlussteil von auf der Aussenseite des Zugbandes aufgebrachten, sich an das erste Verschlussteil anschliessenden und sich über die Länge des Zugbandes erstreckenden relativ dünnen Polfasern gebildet ist.

4. Tennisarm-Bandage nach den Ansprüchen 1 bis 3, dadurch gekennzeichnet, dass das zweite Zugband (120) mittels eines Klettenverschlusses auf der Aussenfläche (10c) der schlauchförmigen Umhüllung (10) lösbar angeordnet ist, und dass zur Ausbildung des Klettenverschlusses das Zugband (120) auf seiner der Aussenfläche (10c) der Umhüllung (10) zugekehrten Fläche einen Abschnitt (126) aus im Abstand voneinander angeordneten, aufwärts gerichteten Häkchen aufweist, während die Umhüllung (10) auf ihrer Aussenfläche (10c) im Bereich des Abschnittes (126) einen in Umhüllungslängsrichtung verlaufenden Abschnitt (16) aus einer Gruppe relativ dünner Polfasern trägt.

5. Tennisarm-Bandage nach den Ansprüchen 1 bis 4, dadurch gekennzeichnet, dass die beiden Zugbänder (20, 120) an ihren die Schnallen (21, 121) tragenden Enden auf der Umhüllung (10) befestigt sind.

## Claims

1. Tennis-elbow bandage (100) which surrounds the forearm over its entire length and which consists of a tubular wrapping (10) made of a transversely and longitudinally elastic woven or knitted fabric, characterized in that the wrapping (10) has, adjacent to its end (10a) pointing towards the elbow, a first tie-band (20) of adjustable length which is intended to rest underneath the bend of the elbow on the pain centre and which is joined firmly to the wrapping (10) and, adjacent to its other end (10b) pointing towards the wrist, a second tie-band (120) of variable length which is fastened releasably to the wrapping (10) and at a variable distance from the first tie-band (20).

2. Tennis-elbow bandage according to Claim 1, characterized in that the two tie-bands (20, 120) are arranged on the wrapping (10) so as to form free end portions (11, 12) of the latter.

3. Tennis-elbow bandage according to Claims 1 and 2, characterized in that each tie-band (20, 120) carries at one end a buckle (21, 121) consisting of a rectangular frame body made of plastic, and in that the other free end of the tie-band (20, 120) can be secured to the tie-band (20, 120) itself by means of a burr closure, one closing part of which consists of a portion located at the free end of the tie-band and on its outer surface and composed of small hooks arranged at a distance from one another and directed upwards, whilst the other closing part is formed by relatively thin pile fibres which are attached to the outside of the tie-band and adjoin the first closing part and which extend over the length of the tie-band.

4. Tennis-elbow bandage according to Claims 1 to 3, characterized in that the second tie-band (120) is arranged releasably on the outer surface (10c) of the tubular wrapping (10) by means of a burr closure, and in that, to form the burr closure, the tie-band (120) has, on its surface turned towards the outer surface (10c) of the wrapping (10), a portion (126) composed of small hooks arranged at a distance from one another and directed upwards, whilst the wrapping (10) carries on its outer surface (10c), in the region of the portion (126), a portion (16) extending in the longitudinal direction of the wrapping and composed of a group of relatively thin pile fibres.

5. Tennis-elbow bandage according to Claims 1 to 4, characterized in that the two tie-bands (20, 120) are fastened to the wrapping (10) at their ends carrying the buckles (21, 121).

## Revendications

1. Bandage pour l'épicondylite (100), qui entoure l'avant-bras sur toute sa longueur et qui se compose d'une gaine souple (10) en tissu ou en tricot, élastique en largeur et en longueur, caractérisé en ce que la gaine (10) présente, au voisinage de son extrémité (10a) orientée vers le coude, une première sangle (20) de longueur réglable, destinée à s'appliquer sur le centre de la douleur sous la saignée du bras et solidement attachée à la gaine (10), et, au voisinage de son autre extrémité (10b) orientée vers le poignet, une seconde sangle (120) qui est fixée à la gaine (10) de façon amovible et à une distance variable de la première sangle (20) et qui est variable en longueur.

2. Bandage pour l'épicondylite suivant la revendication 1, caractérisé en ce que les deux sangles (20, 120) sont montées sur la gaine (10) en ménageant sur celle-ci des portions d'extrémité (11, 12).

3. Bandage pour l'épicondylite suivant les revendications 1 et 2, caractérisé en ce que chaque sangle (20, 120) porte, à sa première extrémité libre, une boucle (21, 121) formée à partir d'un cadre rectangulaire en matière plastique, et en ce que l'autre extrémité libre de la sangle (20, 120) peut être attachée à la sangle (20, 120) elle-même au moyen d'un fermoir à picots dont une partie se compose d'une plage comportant des petits crochets dirigés vers le haut et disposés à une certaine distance l'un de l'autre, prévue sur la face extérieure de l'extrémité libre de la sangle tandis que l'autre partie du fermoir est formée de fibres bouclées relativement fines posées sur la face extérieure de la sangle, se raccordant à la première partie du fermoir et s'étendant sur la longueur de la sangle.

4. Bandage pour l'épicondylite suivant les revendications 1 à 3, caractérisé en ce que la seconde sangle (120) est fixée de façon amovible, au moyen d'un fermoir à picots, sur la face extérieure (10c) de la gaine souple (10), et en ce que, pour constituer le fermoir à picots, la sangle (120) porte sur sa face tournée vers la face extérieure (10c) de la gaine (10) une plage (126) garnie de petits crochets dirigés vers le haut et disposés à une certaine distance l'un de l'autre, tandis que la gaine (10) porte à sa surface extérieure (10c) et dans la région de la plage (126) une plage (16) s'étendant selon la direction longitudinale de la gaine et composée d'un groupe de fibres bouclées relativement fines.

5. Bandage pour l'épicondylite suivant les revendications 1 à 4, caractérisé en ce que les deux sangles (20, 120) sont fixées à la gaine (10), à leurs extrémités portant les boucles (21, 121).

FIG. 1

FIG. 2

FIG. 3